# EUROPÄISCHE PATENTSCHRIFT

(11) **EP 2 769 949 B1**
(45) Veröffentlichungstag und Bekanntmachung des Hinweises auf die Patenterteilung: **30.05.2018**
(21) Anmeldenummer: 13000882.4
(22) Anmeldetag: 21.02.2013
(51) Int. Cl.: B65H 43/00, B65H 7/12

(54) **Verfahren zum messtechnischen Unterscheiden von Materialbereichen eines blatt-, bahn- oder bogenartigen Materials sowie Vorrichtung hierzu**
Method for using measurement technology to differentiate between material zones in the form of a sheet, a web or sheet-like material and device for same
Procédé de différenciation, par une technique de mesure, de zones d'un matériau en forme de feuille, de voie ou d'arc et dispositif correspondant

(43) Veröffentlichungstag der Anmeldung: 27.08.2014
(73) Patentinhaber: Pepperl & Fuchs GmbH, 68307 Mannheim (DE)
(72) Erfinder: Schulz, Enrico, 68307 Mannheim (DE)
(74) Vertreter: Schiffer, Axel Martin

(56) Entgegenhaltungen:
- EP-A1- 1 731 455
- EP-A2- 0 894 756
- EP-A2- 2 039 633
- DE-A1-102004 056 710

## Beschreibung

Die vorliegende Erfindung betrifft ein Verfahren zum messtechnischen Unterscheiden von Materialbereichen, nämlich Majoritätsbereichen und Minoritätsbereichen, eines blatt-, bahn- oder bogenartigen Materials nach dem Oberbegriff des Anspruchs 1.

Bei einem gattungsgemäßen Verfahren wird mindestens ein Sensor verwendet, der ein Messsignal mit einer veränderlichen Amplitude aufnimmt. Die Amplitude variiert abhängig von dem in einem räumlichen Erfassungsbereich des Sensors befindlichen und mit dem Sensor untersuchten Materialbereich und die Amplitude des Messsignals des Sensors wird mit einem steuerbaren Verstärker verstärkt. Weiterhin wird das zu untersuchende Material relativ zu dem Sensor so bewegt, dass sich die Majoritätsbereiche im Vergleich zu den Minoritätsbereichen häufiger in dem räumlichen Erfassungsbereich des Sensors befinden. Umgekehrt bedeutet das selbstverständlich, dass sich die Minoritätsbereiche im Vergleich zu dem Majoritätsbereichen weniger häufig in dem räumlichen Erfassungsbereich des Sensors befinden. Die Majoritätsbereiche weisen eine erste Dicke und eine erste Materialzusammensetzung auf und, ganz entsprechend, weisen die Minoritätsbereiche eine von der ersten Dicke verschiedene zweite Dicke und/oder eine von der ersten Materialzusammensetzung verschiedene zweite Materialzusammensetzung auf. Ein gattungsgemäßes Verfahren ist beispielsweise beschrieben in DE 10 2004 056 742.5.

Sensoren für das Erkennen von Materialstärken bei Bahnen- oder Bogenmaterial sollen für sehr unterschiedliche Dicken und sehr unterschiedliche Materialzusammensetzungen des Mediums einsetzbar sein. Angesichts von Driften, beispielsweise
der Temperatur und der relativen Luftfeuchtigkeit sowie weiterer Änderungen verschiedener Umgebungs- und Maschinenparameter ist das im Allgemeinen schwierig zu realisieren. Nötig ist daher ein "Einlernen", auch als "Teach In" bezeichnet, zumindest bei jedem Medienwechsel. Außerdem muss die Empfindlichkeit im Hinblick auf langsame, durch Temperatur-, Luftdruck- und Luftfeuchteänderungen, durch Materialvariationen und/oder andere relativ langsame Vorgänge bewirkte Effekte nachgeführt werden.
Im Stand der Technik existieren zu dieser allgemeinen Aufgabe prinzipiell zwei Lösungsansätze. Zum einen wird eine feste Schaltschwelle im Betrieb verwendet. Diese kann völlig fest sein oder durch Einlernen festgelegt werden. Um den oben beschriebenen Drifteffekten zu begegnen, muss dabei das Sensorsignal vergleichsweise aufwändig aufbereitet werden. Das erfolgt im Allgemeinen durch geeignete Weiterverarbeitung des ursprünglichen Messsignals mit Hilfe von Kennlinien und/oder variablen Verstärkungen. Das ist in vielen Details in DE 10 2004 056 742 beschrieben.
Sodann kann die Schaltschwelle variabel sein und es wird eine feste oder durch Einlernen festgelegte Verstärkung während des Betriebs verwendet. Dieses Nachführen der Schaltschwelle während des Betriebs ist ebenfalls aufwändig.
Gattungsgemäße Verfahren zum Erkennen von Etiketten, Klebestellen, Abrissstellen, Aufreißfäden und ähnliche Materialien sind beispielsweise beschrieben in DE 10 2004 056 710 A1 und EP 1 731 455 A1. Dabei offenbart DE102004056710 ein Verfahren gemäß dem Oberbegriff des Anspruchs 1 Als **Aufgabe** der vorliegenden Erfindung kann angesehen werden, ein weniger aufwändiges Verfahren zum messtechnischen Unterscheiden von Materialbereichen eines blatt-, bahn- oder bogenartigen Materials anzugeben.
Diese Aufgabe wird durch das Verfahren mit den Merkmalen des Anspruchs 1 gelöst. Vorteilhafte Varianten des erfindungsgemäßen Verfahrens werden im Folgenden, insbesondere im Zusammenhang mit den abhängigen Ansprüchen und den Figuren, beschrieben.
Das Verfahren der oben genannten Art ist erfindungsgemäß dadurch weitergebildet, dass in Phasen, in denen mit dem Sensor Majoritätsbereiche untersucht werden, mit dem Verstärker eine Regelung der Amplitude auf einen Sollwert durchgeführt wird und der Sensor ein Signal "Majoritätsbereich" ausgibt, und dass in Situationen, in denen sich die Amplitude hinreichend stark ändert, der Sensor ein Signal "Minoritätsbereich" ausgibt.

Außerdem geht das erfindungsgemäße Verfahren davon aus, dass eine hinreichend starke Änderung der Amplitude vorliegt, wenn die Amplitude um mehr als einen festzulegenden Betrag von dem Sollwert abweicht, auf den die Amplitude in den Phasen, in denen mit dem Sensor Majoritätsbereiche untersucht werden, geregelt wird und schließlich wird erfindungsgemäß in Situationen, in denen der Sensor das Signal "Minoritätsbereich" ausgibt, die Regelung der Amplitude auf den Sollwert deaktiviert.

Als ein erster wesentlicher Gedanke der vorliegenden Erfindung kann erachtet werden, dass Verfahren spezifischer als bisher im Stand der Technik auf das messtechnische Unterscheiden von Materialien auszurichten, bei denen bestimmte Materialbereiche sehr häufig vorkommen und deshalb als Majoritätsbereiche bezeichnet werden und andere Materialbereiche im Vergleich dazu nur selten vorkommen und aus diesem Grund Minoritätsbereiche genannt werden.

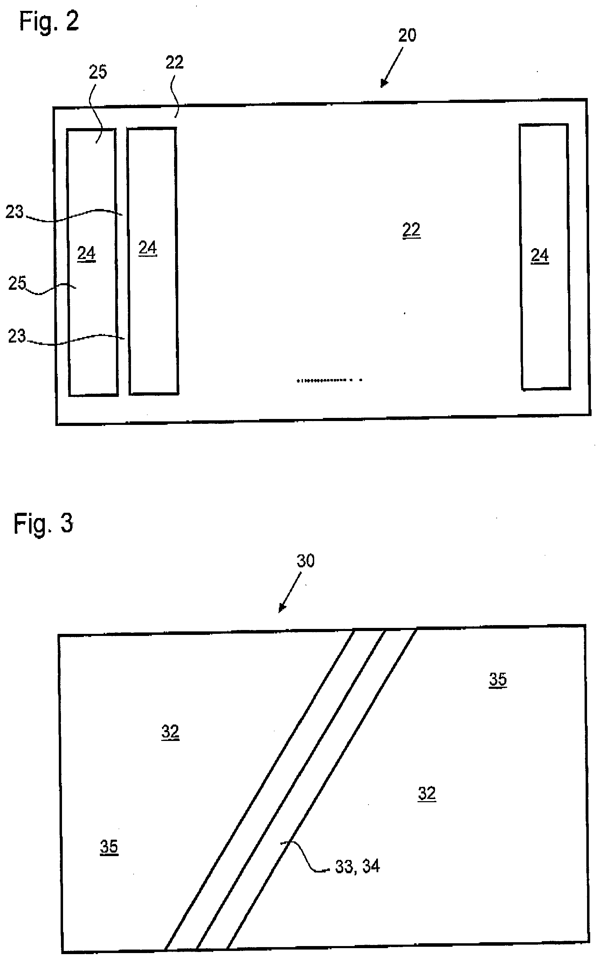

Ein erstes Beispiel hierfür zeigt Fig. 3, wo ein Material (30) mit Papier- oder Folienbahnen als Basismaterial (32) schematisch dargestellt ist. Im gezeigten Beispiel sind zwei Bögen des Basismaterials (32) aneinandergefügt und mit einem Klebestreifen (34) miteinander verbunden. Weil, wie aus Fig. 3 wegen der Flächenverhältnisse sofort ersichtlich, sich das Basismaterial (32) im Vergleich zu dem Klebestreifen (34) viel häufiger im Erfassungsbereich des Sensors befindet, ist in diesem Fall das Basismaterial identisch mit den Majoritätsbereichen (35) und der Klebestreifen stellt die Minoritätsbereiche (33) dar. Solche Materialien werden häufig in der Verpackungstechnik verwendet, wobei mehrere Papier- oder Folienbahnen mithilfe eines Klebestreifens miteinander verbunden sind. Das ist erforderlich, damit eine Verpackungsmaschine beim Wechsel der Papier- oder Folienrollen nicht angehalten werden muss. Weil der Klebestreifen jedoch beim verpackten Endprodukt nicht erwünscht ist, soll der Klebestreifen erkannt und der Maschinensteuerung gemeldet werden, um letzten Endes zu erreichen, dass der Bereich um die Klebestelle aussortiert werden kann.

Ein anderes Beispiel für ein Material, bei dem das erfindungsgemäße Verfahren besonders vorteilhaft angewendet werden kann, ist ein Papier- oder Folienbogen mit darauf aufgebrachten Aufklebern. Ein solches Material 11 ist schematisch in Fig. 2 dargestellt. Auf einem Papier- oder Folienbogen (20), der in diesem Fall das Basismaterial (22) darstellt, sind mit geringen Abständen viele Aufkleber (24) aufgebracht.

Die Aufkleber können auch als Etiketten oder Labels bezeichnet werden. Weil sich die Aufkleber (24) viel häufiger im räumlichen Erfassungsbereich des Sensors, kurz auch nur als Erfassungsbereich bezeichnet, befinden, sind in dieser Situation die Aufkleber (24) die Majoritätsbereiche und entsprechend stellen die Bereiche des Basismaterials (22) zwischen den Aufklebern (24) die Minoritätsbereiche dar.
Ein weiterer Kerngedanke der Erfindung besteht darin, ausgehend von der Erkenntnis, dass eigentlich das Verfahren nur für bestimmte Typen eines blatt-, bahn- oder bogenartigen Materials verwendet werden soll, nämlich denjenigen, wie oben erläutert, die Majoritäts- und Minoritätsbereiche aufweisen, die Amplitude eines Messsignals auf einen Sollwert einzuregeln und dann, vereinfacht gesprochen, zu warten, bis sich die Amplitude des Messsignals deutlich oder, in der Formulierung des Patentanspruchs 1, "hinreichend stark" ändert und dann davon auszugehen, dass sich nun ein Minoritätsbereich des untersuchten Materials im Erfassungsbereich des Sensors befindet.
Ausführliche Tests und Untersuchungen haben ergeben, dass dieses Messprinzip äußerst zuverlässig bei den typischerweise zu prüfenden Materialien einsetzbar ist. Als erster wesentlicher Vorteil der Erfindung kann angesehen werden, dass keinerlei Einlernen des Sensors erforderlich ist. Hieraus ergeben sich weitere Vorteile, nämlich ein besonders einfacher Aufbau mit wenigen Komponenten.
Ein weiterer wesentlicher Vorteil der Erfindung ist, dass langsame Amplitudenänderungen aufgrund von sich ändernden Umgebungsbedingungen, wie Temperatur, Luftdruck und Luftfeuchtigkeit und andere langsam veränderliche Parameter, durch das hier vorgeschlagene Regelungsprinzip kontinuierlich ausgeglichen werden. Prinzipiell kann die Regelung auf den Sollwert ständig eingeschaltet bleiben, insbesondere wenn sich die Minoritätsbereiche nur sehr kurz im Erfassungsbereich des Sensors befinden.
Der Einsatzbereich des erfindungsgemäßen Verfahrens wird deutlich erweitert weil in Situationen, in denen der Sensor das Signal "Minoritätsbereich" ausgibt, die Regelung der Amplitude auf den Sollwert deaktiviert wird. Hierdurch wird erreicht, dass das Verfahren auch dann zuverlässig arbeitet, wenn die Vorschubgeschwindigkeiten des Basismaterials, beispielsweise eines Papiers oder einer Folie, nur gering sind und demgemäß sich eine Klebestelle oder ein Zwischenbereich zwischen zwei Aufklebern vergleichsweise lange im Erfassungsbereich des Sensor befindet. Ohne Abschalten der Regelung würde diese die Amplitude wieder auf den Sollwert regeln. Beispielsweise würde die Klebestelle dann wieder als Basismaterial erkannt werden, was nicht richtig ist. Für diese Fälle wird deshalb die Regelung deaktiviert, sobald der Sensor das Signal "Minoritätsbereich" ausgibt. Erreicht wird dies dadurch, dass die Regelung die Amplitude des Messsignals erfindungsgemäß überwacht und die Regelung entsprechend einstellt.

In diesem Zusammenhang ist außerdem bevorzugt, wenn in Situationen, in denen die Regelung deaktiviert ist und in denen sich die Amplitude hinreichend stark in die entgegengesetzte Richtung wie bei der letzten zurückliegenden Änderung, die zum Deaktivieren der Regelung geführt hat, verändert, die Regelung wieder aktiviert wird und der Sensor das Signal "Majoritätsbereich" ausgibt. Eine zuverlässige Fortführung des Verfahrens wird so sichergestellt.

Prinzipiell kann die Eigenschaft einer "hinreichend starken Änderung" auf grundsätzlich beliebige Art definiert werden. Besonders einfach gestaltet sich das Verfahren, wenn man erfindungsgemäß davon ausgeht, dass eine hinreichend starke Änderung der Amplitude vorliegt, wenn die Amplitude um mehr als einen festzulegenden Betrag von dem Sollwert abweicht, auf den die Amplitude in den Phasen, in denen mit dem Sensor Majoritätsbereiche untersucht werden, geregelt wird.

Ganz entsprechend kann unter einer "hinreichend starken Änderung" der Amplitude in Phasen, in denen der Sensor das Signal "Minoritätsbereiche" ausgibt davon ausgegangen werden, dass eine solche "hinreichend starke Änderung" der Amplitude vorliegt, wenn die Amplitude um weniger als einen festzulegenden Betrag von dem Sollwert abweicht, auf den die Amplitude in den Phasen, in denen mit dem Sensor Majoritätsbereiche untersucht werden, geregelt wird.

Mit anderen Worten wird eine Amplitudenänderung dann als "hinreichend stark" qualifiziert, wenn sie sich, ausgehend von den Phasen, in denen der Sensor "Minoritätsbereich" signalisiert, in ein geeignetes Intervall um den Sollwert bewegt oder, für die Fälle, in denen der Sensor "Majoritätsbereich" signalisiert, sich aus einem geeignet zu wählenden Intervall um den Sollwert herausbewegt.

Damit das Verfahren sofort nach Zuführen eines zu untersuchenden Materials begonnen werden kann, ist es zweckmäßig, wenn der Sensor über eine Sensorkonfiguration oder über einen Steuereingang vor Beginn der Messung eine Information erhält, ob die Amplitude des Messsignals beim Übergang von einem Majoritätsbereich zu einem Minoritätsbereich absinkt oder ansteigt,

Das erfindungsgemäße Verfahren kann besonders vorteilhaft eingesetzt werden, wenn das zu untersuchende Material ein Papierbogen oder Folienbogen mit darauf aufgebrachten Klebestreifen oder Klebestellen ist oder wenn das zu untersuchende Material ein Papier- oder Folienbogen mit darauf aufgebrachten Aufklebern ist.

Diese beiden Materialien werden in der Verpackungs- und Automatisierungstechnik häufig verwendet.

Die Regelung der Amplitude auf den Sollwert in Phasen, in denen Majoritätsbereiche mit dem Sensor untersucht werden, kann auf prinzipiell bekannte Weise, beispielsweise mithilfe eines PID-Reglers, durchgeführt werden. Eine einfache und deshalb besonders bevorzugte Verfahrensvariante ist dadurch gekennzeichnet, dass die Regelung der Amplitude auf den Sollwert durchgeführt wird, indem ein Mittelwert einer Anzahl von zurückliegenden Messungen der Amplitude bestimmt wird, und dass, wenn der Mittelwert über dem Sollwert liegt, die Verstärkung des Verstärkers um eine Einheit verringert wird und die Regelung mit dieser Verstärkung fortgesetzt wird und dass, wenn der Mittelwert unter dem Sollwert liegt, die Verstärkung um eine Einheit erhöht wird und die Regelung mit dieser Verstärkung fortgesetzt wird.

Eine solche Regelung hat gegenüber einem klassischen Proportional-Integral-Differenzial-Regler (PID-Regler) den Vorteil, dass weniger Rechenaufwand benötigt wird und dass diese Regelung prinzipiell unabhängig von der Stärke der Amplitudenschwankungen ist.

Einen Sonderfall für die Regelung stellt eine Situation dar, in der das geprüfte Material, beispielsweise also ein Papier- oder Folienbogen, gewechselt wird. Nicht selten wird an einer Maschine beispielsweise mehrmals am Tag das Papier gewechselt. In diesem Zusammenhang ist eine Verfahrensvariante zweckmäßig und bevorzugt, bei der in Situationen, in denen die Amplitude über eine festzulegende Zeitspanne einen größtmöglichen Wert annimmt, der Sensor davon ausgeht, dass sich kein Material im räumlichen Erfassungsbereich des Sensors befindet. Der Verstärker wird dann auf die kleinstmögliche Verstärkung gesteuert. Die Regelung bleibt aktiv und versucht die Amplitude auf den Sollwert zu regeln. Das gelingt ihr aber nicht, weil die Amplitude selbst bei der geringstmöglichen Verstärkung deutlich über dem Sollwert liegt. Dieser Modus wird erst wieder verlassen, wenn die Amplitude absinkt, insbesondere also dann, wenn die Amplitude des Messsignals um einen festzulegenden Betrag von der maximalen Amplitude abweicht. Das ist zum Beispiel der Fall, wenn wieder ein Material in den Erfassungsbereich des Sensors gebracht wird.

Mit dieser Verfahrensvariante ist es möglich, zu erkennen, wenn sich für eine gewisse Zeit kein Material im räumlichen Erfassungsbereich des Sensors befindet. Nach Ablauf dieser Zeit aktiviert die Regelung einen Papierwechselmodus oder allgemeiner einen Materialwechselmodus.

Als Sensor können prinzipiell alle Sensoren eingesetzt werden, mit denen eine Unterscheidung von Majoritätsbereichen und Minoritätsbereichen der hier diskutierten Materialien möglich ist. Prinzipiell können hierzu auch Lichtschranken oder reflektiertes Licht messende Sensoren eingesetzt werden. Besonders bevorzugt werden aber Ultraschallsensoren oder kapazitive Sensoren verwendet, wobei auch hier Schrankensensoren oder Reflexionssensoren verwendet werden können.

Bei den vorstehend beschriebenen Materialien "Papier mit Klebestreifen" einerseits und "Papier mit Aufklebern" andererseits ist das Basismaterial, also das Papier, im ersten Fall der "Majoritätsbereich", im zweiten Fall aber der "Minoritätsbereich". Um hierdurch möglicherweise entstehende Unklarheiten für einen Benutzer zu vermeiden, ist eine Verfahrensvariante zweckmäßig, bei der außerdem Signale "Klebestelle", "Aufkleber" und/oder "Basismaterial" ausgegeben werden nach Maßgabe folgender Kriterien:
Wenn ein Material untersucht wird, bei dem die Amplitude beim Übergang von den Majoritätsbereichen zu den Minoritätsbereichen sinkt (zum Beispiel bei einer Klebestellenerkennung), wird das Signal "Basismaterial" ausgegeben, wenn der Sensor außerdem das Signal "Majoritätsbereich" ausgibt und es wird bei einem solchen Material das Signal "Klebestelle" ausgegeben, wenn der Sensor außerdem das Signal "Minoritätsbereich" ausgibt. Andererseits wird, wenn ein Material untersucht wird, bei dem die Amplitude beim Übergang von den Majoritätsbereichen zu den Minoritätsbereichen steigt (beispielsweise bei der Amplitudenerkennung), das Signal "Basismaterial" ausgegeben, wenn der Sensor außerdem "Minoritätsbereich" ausgibt und es wird bei diesem Material das Signal "Aufkleber" ausgegeben, wenn der Sensor außerdem das Signal "Majoritätsbereich" ausgibt.

Unter den Vorgängen "Ausgeben" und gleichbedeutend damit "Signalisieren" soll für die vorliegende Erfindung ganz allgemein ein Bereitstellen eines entsprechenden Signals verstanden werden, beispielsweise können diese Signale "Majoritätsbereich", "Minoritätsbereich", "Basismaterial", "Klebestellen", "Aufkleber" "Materialwechsel" an, insbesondere separaten, Ausgängen des Sensors bereitgestellt werden. Diese Informationen können dann von einer übergeordneten Einheit verwendet werden, um sie einem Benutzer zugänglich, insbesondere optisch zugänglich, zu machen.

Weitere Vorteile, Merkmale und Eigenschaften der vorliegenden Erfindung werden im Folgenden mit Bezug auf die beiliegenden Figuren beschrieben. Hierin zeigt:
- Fig. 1:: in schematischer Ansicht einen zur Durchführung des erfindungsgemäßen Verfahrens geeigneten Messaufbau;
- Fig. 2:: ein erstes Material, welches zur Untersuchung mithilfe des erfindungsgemäßen Verfahrens geeignet ist;
- Fig. 3:: ein zweites Material, welches zur Untersuchung mit dem erfindungsgemäßen Verfahren geeignet ist;
- Fig. 4:: eine schematische Veranschaulichung einer bei dem erfindungsgemäßen Verfahren einsetzbaren Regelung sowie einer Variante einer für das erfindungsgemäße Verfahren einsetzbaren Auswertung;
- Fig. 5:: in schematischer Darstellung ein Ablaufdiagramm des erfindungsgemäßen Verfahrens für ein Material, bei dem die Amplitude des Messwerts bei einem Übergang von einem Majoritätsbereich zu einem Minoritätsbereich sinkt; und
- Fig. 6:: ein Diagramm, welches die Amplitude des Messsignals aufgetragen gegen die Zeit für die im Zusammenhang mit Fig. 5 beschriebene Messsituation darstellt.

Gleiche oder gleich wirkende Komponenten und Bestandteile sind in den Figuren in der Regel mit denselben Bezugszeichen gekennzeichnet.

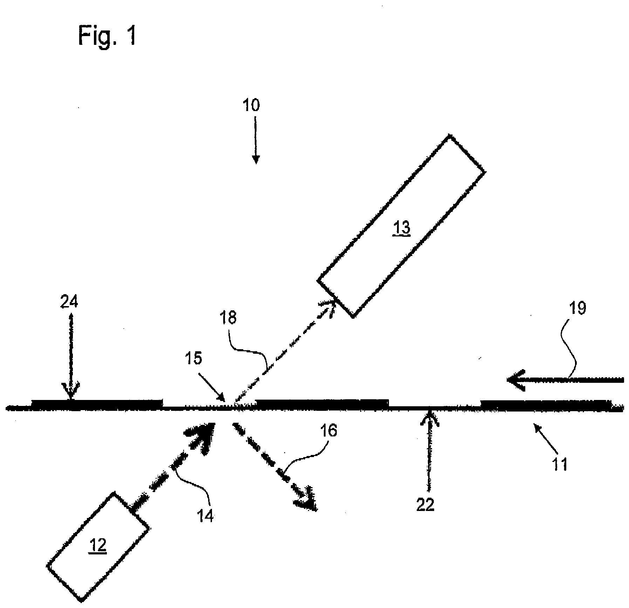

Die grundlegende Messsituation wird anhand von Fig. 1 erläutert. Dargestellt ist dort ein Schrankensensor 10, der einen Sender 12 und einen Empfänger 13 aufweist. Es handelt sich bei Fig. 1 um eine schematische Darstellung. In Wirklichkeit enthält der Sensor 10 noch weitere Komponenten, insbesondere einen Mikrocontroller oder ein vergleichbares Bauteil, welches die Messaufnahme und die Auswertung steuert. Nicht dargestellt ist hier auch der erfindungsgemäß vorgesehene einstellbare Verstärkerteil des Sensors 10. In dem in Fig. 1 gezeigten Beispiel handelt es sich um einen Ultraschallsensor, das heißt der Sender 12 emittiert Ultraschallwellen 14 in Richtung eines zu untersuchenden Materials 11, wobei es sich im gezeigten Beispiel um eine Papier- oder Folienbahn 22 handelt. Die von dem Sender 12 emittierte Ultraschallstrahlung 14 trifft in einem räumlichen Erfassungsbereich 15 des Sensors auf das Material 11 und wird dort reflektiert und transmittiert. Der reflektierte Anteil des Ultraschalls ist in Fig. 1 durch einen Pfeil 16 dargestellt. Ein Pfeil 18, der auf den Empfänger 13 trifft, stellt den transmittierten und gedämpften Anteil des Ultraschalls dar. In der in Fig. 1 gezeigten Situation befindet sich ein Teil des Papier- oder Folienbogens 22, der hier auch als Basismaterial 22 bezeichnet wird, im Erfassungsbereich 15 des Sensors. Auf dem Basismaterial 22 sind Aufkleber 24 aufgebracht. Ziel der Messung ist es, festzustellen, ob sich ein Teil des Basismaterials 22 oder ein Aufkleber 24 im räumlichen Erfassungsbereich 15 des Sensors 10 befindet. Hierzu werden der Sensor 10 und das Material 11 kontinuierlich relativ zueinander bewegt, was in Fig. 1 durch den Pfeil 19 veranschaulicht ist.

An den Stellen, wo sich, wie in Fig. 1 gezeigt, ein Aufkleber 24 oder eine Klebestelle (in Fig. 1 nicht dargestellt) auf dem Material befindet, werden die Ultraschallwellen noch stärker gedämpft, so dass die Amplitude des Messsignals des Empfängers 13 abnimmt. Kernidee der Erfindung ist es, den Aufkleber 24 oder einen Klebestreifen anhand dieser Amplitudenänderung nachzuweisen.

Fig. 1 zeigt demgemäß einen Sensor, mit dem das Vorhandensein von Aufklebern 24 auf einem Basismaterial 22 nachgewiesen werden kann. Dabei muss der Sensor 10 auf die Lücke zwischen zwei Aufklebern 24 reagieren. Weil diese sehr klein sein können, ist eine kurze Ansprechzeit von deutlich unterhalb einer Millisekunde erforderlich.

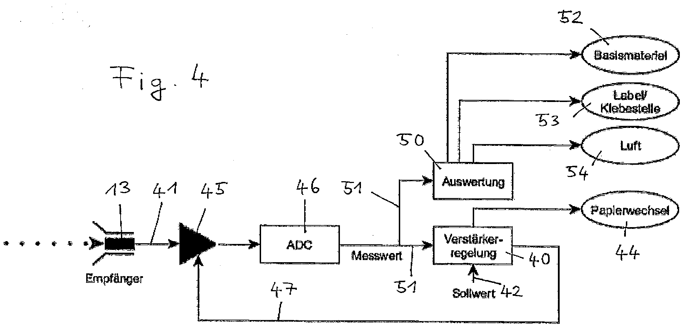

Fig. 4 veranschaulicht in schematischer Form elementare Abläufe bei der Regelung des Verstärkers. Diese Regelung ist dort schematisch als Kasten 40 dargestellt. Die Steuerung des Verstärkers 45 durch die Regelung 40 ist durch einen Pfeil 47 veranschaulicht. Als Eingangsdaten werden in dem Verstärker 45 eine tatsächlich gemessene Amplitude (Pfeil 41) des Messsignals des verwendeten Sensorempfängers 13 zugeführt. Der Regelung 40 wird außerdem ein Sollwert 42 zugeführt, auf den die Amplitude geregelt werden soll, wenn sich ein Majoritätsbereich 25, 35 im Erfassungsbereich 15 des Sensors 10 befindet. Die Verstärkung des Verstärkers 45 hängt ab von einer Einstellung eines hier nicht gezeigten Digitalpotentiometers. Die Regelung 40 stellt das Digitalpotentiometer den eingestellten Parametern der Regelung entsprechend ein.

Wie vorstehend im Einzelnen beschrieben, kann der Verstärker aktiv sein, typischerweise dann, wenn Majoritätsbereiche untersucht werden oder der Verstärker kann auch deaktiviert sein, was beispielsweise der Fall ist, wenn sich nichts im räumlichen Erfassungsbereich des Sensors befindet oder wenn ein Minoritätsbereich untersucht wird.

Weil der Zustand "Papierwechsel" durch eine minimale Verstärkung charakterisiert ist, kann dieser Zustand direkt an der Regelung 40 abgefragt oder von der Regelung 40 ausgegeben werden. Die Ausgabe des Zustands "Papierwechsel" ist in Fig. 4 mit dem Bezugszeichen 44 gekennzeichnet.

Die Verstärkung kann über das Digitalpotentiometer per Software eingestellt werden. Die Regelung 40 erhält dabei, wie in Fig. 4 schematisch dargestellt, nach jeder Analog-Digital-Wandlung des Analog-Digital-Wandlers 46 einen aktuellen Messwert und einen Sollwert 42, auf den geregelt werden soll. Ziel ist es, die Verstärkung so einzuregeln, dass der Mittelwert der Amplitude über mehrere Messwerte auf dem vorgegebenen Sollwert liegt. Als Regelalgorithmus kann beispielsweise eine einfache Addition oder Subtraktion der Verstärkung zum Einsatz kommen. Das bedeutet: Wenn der Mittelwert über dem Sollwert liegt, wird die Verstärkung um eine Einheit oder ein Digit verringert. Liegt der Mittelwert andererseits darunter, so wird die Verstärkung um eine Einheit oder ein Digit erhöht.

Die Regelung darf dabei nicht zu schnell erfolgen, weil die Einbrüche der Amplitude, beispielsweise an einer Klebestelle, nicht zu schnell ausgeregelt werden dürfen. Andererseits darf die Regelung aber auch nicht zu langsam sein, weil, wie oben erläutert, der Sensor eine Ansprechzeit von maximal einer Sekunde haben darf.

Fig. 4 zeigt außerdem in abstrahierter Form Merkmale der Auswertung 50, in Fig. 4 schematisch durch einen Kasten dargestellt. Die Auswertung 50 beinhaltet Verfahrensschritte des erfindungsgemäßen Verfahrens nach Aufnahme der Messdaten mit dem Sensor 10. Diese Messdaten, prinzipiell also die Amplitude des Messsignals, werden einer Auswertung 50 zugeführt, in Fig. 4 veranschaulicht durch den Pfeil 51. Die Auswertung 50 wertet diese Daten nach Maßgabe von bereits vorgenommenen Einstellungen aus und gibt nach Maßgabe der weiteren erfindungsgemäß vorgesehenen Verfahrensschritte Signale aus. In dem in Fig. 4 gezeigten Beispiel sind das die Signale "Basismaterial" 52, "Label/Klebestelle" 53 und "Luft" 54, was dasselbe bedeutet wie "Papierwechsel" oder "Materialwechsel".

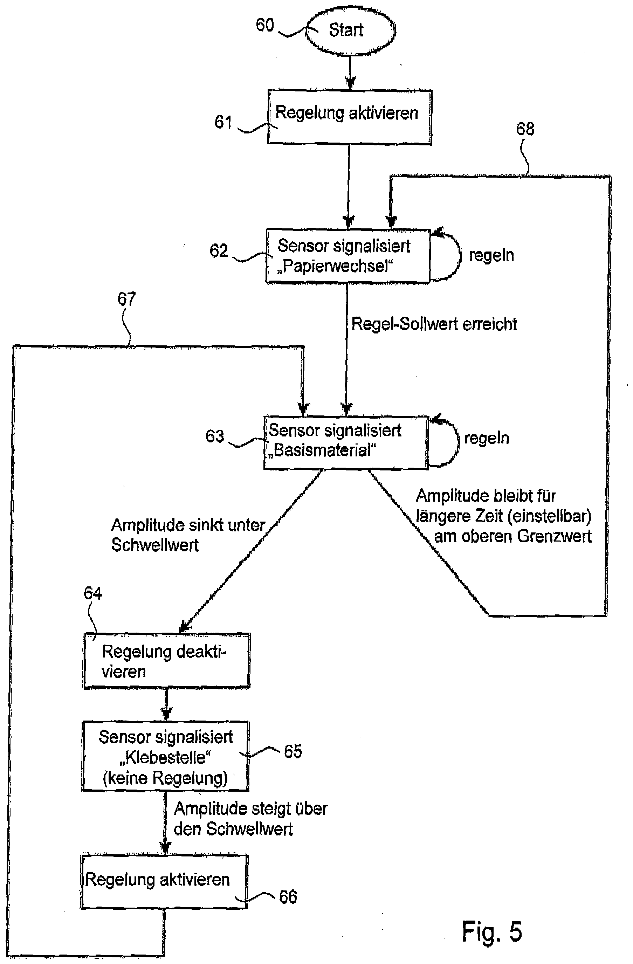

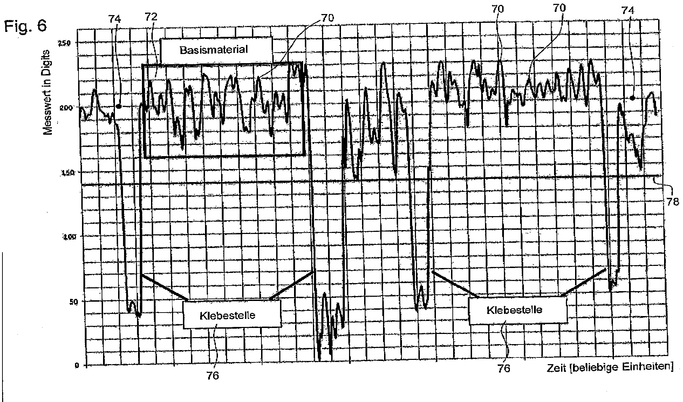

Der Ablauf des erfindungsgemäßen Verfahrens wird im Folgenden anhand der Figuren 5 und 6, näher erläutert. Dargestellt ist in den Figuren 5 und 6 eine Verfahrensvariante, bei der Klebestellen erkannt werden sollen. In Fig. 6 ist ein Verlauf einer Amplitude 70 des Messsignals in beliebigen Einheiten (Digits) gegen die Zeit (ebenfalls in beliebigen Einheiten) aufgetragen. Dargestellt ist in Fig. 6 außerdem ein Sollwert 74 und eine Schwelle 78.

Bei Sensoren aus dem Stand der Technik werden feste Schaltschwellen definiert. Unterschreitet die Messamplitude diese Schwelle, schaltet der Sensor einen Ausgang. Weil jedes Papier unterschiedlich stark dämpft, muss bei diesen Sensoren die von dem jeweiligen Basismaterial, beispielsweise also Papier, bewirkte Messamplitude eingelernt werden und die Schaltschwellen werden dann typischerweise 30% oberhalb oder unterhalb dieses Signals festgelegt, je nachdem, ob Aufkleber oder Klebestellen erkannt werden soll.

Weil bei dem erfindungsgemäßen Verfahren die Verstärkung durch eine Regelung gesteuert wird, liegt die Amplitude des Messsignals unabhängig von dem untersuchten Material 11 immer auf demselben Wert. Als bedeutender Vorteil der vorliegenden Erfindung wird deshalb erreicht, dass ein Einlernen nicht mehr nötig ist. Beispielsweise, wie in Fig. 6 gezeigt, kann die Amplitude 70 des Messsignals auf einen Wert von 200 Digit als Sollwert 74 geregelt werden.

Der Mittelwert der Amplitude 70 ist in Phasen, in denen die Majoritätsbereiche 35 des Materials 30 (siehe Fig. 3) untersucht werden, auf 200 Digit ausgeregelt. Beispielhaft ist das in Fig. 6 im Kasten 72 gezeigt, wo die Amplitude 70 um den Wert 200 Digit hin- und herschwankt. In Fig. 6 sind außerdem vier Bereiche zu erkennen, wo die Amplitude 70 unter die Schwelle 78, durch welche in diesem Fall eine hinreichend starke Änderung im Vergleich zu dem Sollwert 74 gekennzeichnet ist, absinkt. Diese vier Bereiche sind durch die beiden Kästen 76 kenntlich gemacht.

Die bei festen Schaltschwellen entstehenden Probleme werden deutlich an Stellen, wo der Verlauf der Amplitude 70. der Schwelle 78 sehr nahe kommt, diese aber nicht unterschreitet. Grund für diese sehr niedrigen Werte der Amplitude 70 sind vermutlich Inhomogenitäten des Materials 30. Es besteht demgemäß an diesen Stellen ein großes Risiko, dass der Sensor falsch schaltet.

Der Ablauf des erfindungsgemäßen Verfahrens wird anhand von Fig. 5 für das Beispiel einer Klebestellenerkennung beschrieben.

Nach einem Start 60 des Sensors wird zunächst die Regelung aktiviert, der Sensor signalisiert in einem Verfahrensschritt 62 "Materialwechsel" oder "Papierwechsel" und versucht gleichzeitig auf einen dem Sensor zuvor mitgeteilten oder jedenfalls bekannten Sollwert zu regeln. Solange sich kein Material im Erfassungsbereich des Sensors befindet, wird diese Regelung nicht möglich sein, weil bereits bei der geringstmöglichen Verstärkung die Amplitude 70 des Signals oberhalb des Sollwerts 74 liegen wird. Erst wenn ein zu untersuchendes Material 30 (siehe Fig. 3) in den Erfassungsbereich 15 des Sensors 10 eingebracht wird, ist diese Regelung auf den Sollwert möglich und der Sollwert 74 kann erreicht werden. Der Sensor gibt dann im Verfahrensschritt 63 das Signal "Basismaterial" aus. Hieran ändert sich solange nichts, bis die Amplitude 70 des Messsignals entweder unter den Schwellwert 78 sinkt und deshalb, mit den Worten des Anspruchs 1, eine hinreichend starke Änderung der Amplitude vorliegt. Dies führt zum Verfahrensschritt 64, in dem die Regelung deaktiviert wird. Die Verstärkung kann dabei auf dem zuletzt eingenommenen Wert beibehalten werden. Der Sensor gibt dann das Signal "Klebestelle" aus (Schritt 65 in Fig. 5).

Wichtig ist hierbei, dass die Regelung bei den Majoritätsbereichen 25, 35 (siehe Figuren 2 und 3) erfolgt, das heißt den Materialbereichen, die sich häufiger im Erfassungsbereich des Sensors befinden als andere Materialbereiche. Bei einer Klebestellenerkennung sind die Majoritätsbereiche, wie aus Fig. 3 ersichtlich, die Bereiche des Basismaterials 32. Andererseits sind bei einer Aufklebererkennung die Aufkleber 24 selbst die Majoritätsbereiche 25 (siehe Fig. 2).

Im Fall der Prüfung eines Materials 20, bei dem Aufkleber 24 auf einem Basismaterial 22 aufgebracht sind, wie in Fig. 2, würde die Amplitude, ausgehend von Schritt 63, wo der Sensor ein Signal "Aufkleber" ausgeben würde, ansteigen.

In dem in Fig. 5 gezeigten Beispiel folgt nach Schritt 64 der Schritt 65, wo der Sensor das Signal "Klebestelle" ausgibt und die Regelung deaktiviert ist.

Im Fall einer Aufklebererkennung würde hier der Sensor statt des Signals "Klebestelle" das Signal "Basismaterial" ausgeben.

Zurück zur Fig. 5: Sobald die Amplitude 70 des Messsignals wieder über den Schwellwert 78 steigt, wird die Regelung in Schritt 66 wieder aktiviert und das Verfahren kehrt, angedeutet durch den Pfeil 67, zurück zum Schritt 63, wo der Sensor das Signal "Basismaterial" ausgibt. Die Schritte 64, 65 und 66 werden demgemäß jedes Mal durchlaufen, wenn sich ein Minoritätsbereich im Erfassungsbereich des Sensors befindet. Das ist in dem in Fig. 5 gezeigten Beispiel eine Klebestelle und wäre im Fall einer Aufklebererkennung ein kleiner Bereich des Basismaterials zwischen den Aufklebern.

Einen Sonderfall, der gleichwohl häufig auftreten kann, stellt die Situation eines Material- oder Papierwechsels dar. Beispielsweise muss bei manchen Maschinen das Papier mehrmals am Tag gewechselt werden. Mit dem erfindungsgemäßen Verfahren kann das erkannt werden, weil sich in diesem Fall für eine bestimmte Zeit kein Material im Erfassungsbereich des Sensors befindet. Wenn also die Amplitude sich für eine zu wählende und einzustellende Zeit in der Nähe eines oberen Grenzwerts oder an diesem oberen Grenzwert befindet, geht der Sensor, in Fig. 5 durch einen Pfeil 68 dargestellt, wieder zum Verfahrensschritt 62 über, wo der Sensor "Materialwechsel" oder "Papierwechsel" signalisiert und außerdem versucht, auf den Sollwert 74 einzuregeln. Die Schaltausgänge "Klebestelle" und "Basismaterial" werden deaktiviert und bleiben deaktiviert, solange das Verfahren im Verfahrensschritt 62 verharrt. Signalisiert wird allerdings ein "Papierwechsel" oder "Materialwechsel".

Der Verstärker bleibt dann auf der niedrigsten Verstärkung, so dass sich die Amplitude 70, ohne Material 20, 30 (siehe Figuren 2 und 3) im Erfassungsbereich des Sensors, im Vollausschlag befindet. Aufgrund der niedrigen Verstärkung ist sichergestellt, dass die Amplitude 70 auch bei einem sehr schwach dämpfenden Material sehr stark absinkt. So kann die Regelung sicher erkennen, wenn sich wieder ein Material, beispielsweise Papier, in dem räumlichen Erfassungsbereich des Sensors 10 befindet. Die Amplitude 70 wird dann wieder auf den Sollwert 74 geregelt. Sobald der Sollwert 74 erreicht wurde, geht das Verfahren über zum Verfahrensschritt 63 und es schließen sich die oben bereits beschriebenen Verfahrensschritte an.

Bevorzugt wird ein Sensor verwendet, bei dem alle Einstellungen der Regelung von einem Rechner aus konfiguriert werden können, beispielsweise über eine Infrarotschnittstelle oder grundsätzlich auch über jede andere Schnittstelle.

Mit der vorliegenden Erfindung wird ein neues Verfahren zum Erkennen von Aufklebern einerseits und zum Erkennen von Klebestellen oder Klebestreifen andererseits bereitgestellt. Die Erfindung ist anwendbar auf Materialsensoren, die während des Betriebs nur zwischen zwei unterschiedlichen Mediendicken zu unterscheiden haben, wobei dies aber über einen sehr weiten Bereich der Mediendicke und des Medienmaterials möglich sein soll. Der Wechsel zwischen den beiden Materialbereichen sollte möglichst rasch erfolgen. Deshalb ist die erfindungsgemäße Lösung vor allem für Aufkleber- und Klebestellen-Erkennung gut geeignet. Abstrahiert wird also ein Verfahren bereitgestellt, das eine Unterscheidung von zwei Amplitudenwerten durch einen Schaltausgang ohne Einlernen auf eine der beiden Amplituden ermöglicht.

Dieser rasche Wechsel zwischen den Materialbereichen stellt für den Sensor ein Triggersignal dar, das für den Sensor eine Veränderung des Selbstabgleichs bewirkt: Der Sensor geht von einem Default-Szenario aus und regelt mit variabler Verstärkung die Amplitude des Messsignals auf den Messbereich eines Vergleichers mit einer, insbesondere festen, Schaltschwelle. Dieser Selbstabgleich funktioniert kontinuierlich. Das Default-Szenario bedeutet, dass die Regelung bei den Materialbereichen erfolgt, die sich am häufigsten im Erfassungsbereich des Sensors befinden, nämlich den Majoritätsbereichen. Im Fall der Aufkleber-Erkennung stellen die Aufkleber selbst die Majoritätsbereiche dar. Für die Klebestellen-Erkennung sind, was wegen der Flächenverhältnisse unmittelbar einsichtig ist, die Majoritätsbereiche durch das Papier oder jedenfalls das Basismaterial gegeben.

Kommt es zu einem der oben genannten Wechsel, so wird zunächst die Verstärkung nicht mehr nachgeregelt und der Sensor kann nun zwischen Basismaterial und Klebestelle beziehungsweise zwischen Etikette und Basismaterial unterscheiden. Darauf basierend wird dann der Selbstabgleich wieder kontinuierlich durchgeführt bis zum nächsten Wechsel.

Als besonderer Vorteil der Erfindung wird außerdem erreicht, dass durch den kontinuierlichen Selbstabgleich auch Störeinflüsse zwischen den Wechselvorgängen ausgeblendet werden.

## Patentansprüche

1. Verfahren zum messtechnischen Unterscheiden von Materialbereichen, nämlich Majoritätsbereichen und Minoritätsbereichen, eines blatt-, bahn- oder bogenartartigen Materials, wobei
• mindestens ein Sensor (10) verwendet wird, der ein Messsignal mit einer veränderlichen Amplitude (70) aufnimmt,
• die Amplitude (70) des Messsignals des Sensors (10) abhängig unter anderem von dem in einem räumlichen Erfassungsbereich (15) des Sensors (10) befindlichen und mit dem Sensor (10) untersuchten Materialbereich variiert,
• dass die Amplitude (70) des Messsignals des Sensors (10) mit einem einstellbaren Verstärker (45) verstärkt wird,
• das zu untersuchende Material (11) relativ zu dem Sensor (10) so bewegt wird, dass sich die Majoritätsbereiche (25, 35) im Vergleich zu den Minoritätsbereichen (23, 33) häufiger in dem räumlichen Erfassungsbereich (15) des Sensors (10) befinden,
• die Majoritätsbereiche (25, 35) eine erste Dicke und eine erste Materialzusammensetzung aufweisen,
• die Minoritätsbereiche (23, 33) eine von der ersten Dicke verschiedene zweite Dicke und/oder eine von der ersten Materialzusammensetzung verschiedene zweite Materialzusammensetzung aufweisen,
• in Phasen, in denen mit dem Sensor (10) Majoritätsbereiche (25, 35) untersucht werden, mit dem Verstärker (45) eine Regelung der Amplitude (70) auf einen Sollwert (74) durchgeführt wird und der Sensor (10) ein Signal "Majoritätsbereich" ausgibt, und
• in Situationen, in denen sich die Amplitude (70) hinreichend stark ändert, der Sensor (10) ein Signal "Minoritätsbereich" ausgibt,
**dadurch gekennzeichnet,**
**dass** eine hinreichend starke Änderung der Amplitude (70) vorliegt, wenn die Amplitude (70) um mehr als einen festzulegenden Betrag von dem Sollwert (74) abweicht, auf den die Amplitude (70) in den Phasen, in denen mit dem Sensor (10) Majoritätsbereiche (25, 35) untersucht werden, geregelt wird, und dass in Situationen, in denen der Sensor (70) das Signal "Minoritätsbereich" ausgibt, die Regelung der Amplitude (70) auf den Sollwert (74) deaktiviert wird.

2. Verfahren nach Anspruch 1,
**dadurch gekennzeichnet,**
**dass** in Situationen, in denen die Regelung deaktiviert ist und in denen sich die Amplitude (70) hinreichend stark in die entgegengesetzte Richtung wie bei der letzten zurückliegenden Änderung, die zum Deaktivieren der Regelung geführt hat, verändert, die Regelung wieder aktiviert wird und der Sensor das Signal "Majoritätsbereich" ausgibt.

3. Verfahren nach Anspruch 2,
**dadurch gekennzeichnet,**
**dass** eine hinreichend starke Änderung der Amplitude (70) vorliegt, wenn die Amplitude (70) um weniger als einen festzulegenden Betrag von dem Sollwert (74) abweicht, auf dem die Amplitude (70) in den Phasen, in denen mit dem Sensor (10) Majoritätsbereiche (25, 35) untersucht werden, geregelt wird.

4. Verfahren nach einem der Ansprüche 1 bis 3,
**dadurch gekennzeichnet,**
**dass** der Sensor (10) über eine Sensorkonfiguration oder über einen Steuereingang vor Beginn der Messung eine Information erhält, ob die Amplitude (70) des Messsignals beim Übergang von einem Majoritätsbereich (25, 35) zu einem Minoritätsbereiche (23, 33) absinkt oder ansteigt.

5. Verfahren nach einem der Ansprüche 1 bis 4,
**dadurch gekennzeichnet,**
**dass** das zu untersuchende Material (11) ein Papier- oder Folienbogen (32) mit darauf aufgebrachten Klebestreifen (34) oder Klebestellen ist oder dass das zu untersuchende Material (11) ein Papier- oder Folienbogen (22) mit darauf aufgebrachten Aufklebern (24) ist.

6. Verfahren nach einem der Ansprüche 1 bis 5,
**dadurch gekennzeichnet,**
**dass** die Regelung der Amplitude (70) auf den Sollwert (74) durchgeführt wird, indem ein Mittelwert einer Anzahl von zurückliegenden Messungen der Amplitude (70) bestimmt wird, und
**dass**, wenn der Mittelwert über dem Sollwert (74) liegt, die Verstärkung um eine Einheit verringert wird und die Regelung mit dieser Verstärkung fortgesetzt wird und
**dass**, wenn der Mittelwert unter dem Sollwert (74) liegt, die Verstärkung um eine Einheit erhöht wird und die Regelung mit dieser Verstärkung fortgesetzt wird.

7. Verfahren nach einem der Ansprüche 1 bis 6,
**dadurch gekennzeichnet,**
**dass** in Situationen, in denen die Amplitude (70) über eine festzulegende Zeitspanne einen größtmöglichen Wert annimmt, der Sensor (10) davon ausgeht, dass sich kein Material (11) im räumlichen Erfassungsbereich (15) des Sensors (10) befindet.

8. Verfahren nach einem der Ansprüche 1 bis 7,
**dadurch gekennzeichnet,**
**dass** Signale "Klebestelle", "Aufkleber" und "Basismaterial" nach folgender Maßgabe von dem Sensor (10) ausgegeben werden:
a) wenn ein Material (30) untersucht wird, bei dem die Amplitude (70) beim Übergang von den Majoritätsbereichen (35) zu den Minoritätsbereichen (33) sinkt, wird
a1) das Signal "Basismaterial" ausgegeben, wenn der Sensor (10) außerdem das Signal "Majoritätsbereich" ausgibt; und
a2) das Signal "Klebestelle" ausgegeben, wenn der Sensor (10) außerdem das Signal "Minoritätsbereich" ausgibt;
b) wenn ein Material (20) untersucht wird, bei dem die Amplitude (70) beim Übergang von den Majoritätsbereichen (25) zu den Minoritätsbereichen (23) steigt, wird
b1) das Signal "Basismaterial" ausgegeben, wenn der Sensor (10) außerdem das Signal "Minoritätsbereich" ausgibt; und
b2) das Signal "Aufkleber" ausgegeben, wenn der Sensor (10) außerdem das Signal "Majoritätsbereich" ausgibt.

9. Verfahren nach einem der Ansprüche 1 bis 8,
**dadurch gekennzeichnet,**
**dass** als Sensor (10) ein kapazitiver Sensor oder ein Ultraschallsensor verwendet wird.

10. Verfahren nach einem der Ansprüche 1 bis 9,
**dadurch gekennzeichnet,**
**dass** ein Schrankensensor oder ein Reflexionssensor verwendet wird.

## Claims

1. Method for differentiating between measurements of material zones, namely majority zones and minority zones, of a sheet-like or web-like material, wherein
- at least one sensor (10) is used, which records a measurement signal having a varying amplitude (70),
- the amplitude (70) of the measurement signal of the sensor (10) varies in dependence on, inter alia, the material zone located in a spatial detection region (15) of the sensor (10) and examined with the sensor (10),
- the amplitude (70) of the measurement signal of the sensor (10) is amplified with an adjustable amplifier (45),
- the material (11) to be examined is moved relative to the sensor (10) so that the majority zones (25, 35) are located in the spatial detection region (15) of the sensor (10) more frequently than the minority zones (23, 33),
- the majority zones (25, 35) have a first thickness and a first material composition,
- the minority zones (23, 33) have a second thickness that differs from the first thickness and / or a second material composition that differs from the first material composition,
- in phases in which majority zones (25, 35) are examined with the sensor (10), an adjustment of the amplitude (70) to a target value (74) is carried out with the amplifier (45), and the sensor (10) outputs a "majority zone" signal, and
- in situations in which the amplitude (70) changes sufficiently greatly the sensor (10) outputs a "minority zone" signal,
**characterised in that**
a sufficiently great change in the amplitude (70) is present if the amplitude (70) deviates by more than an amount to be fixed from the target value (74), to which the amplitude (70) is adjusted in the phases in which majority zones (25, 35) are examined with the sensor (10), and in situations in which the sensor (10) outputs the "minority zone" signal, the adjustment of the amplitude (70) to the target value (74) is deactivated.

2. Method according to claim 1,
**characterised in that**
in situations in which the adjustment is deactivated and in which the amplitude (70) changes sufficiently greatly in the opposite direction as in the most recent change that led to the deactivation of the adjustment, the adjustment is reactivated and the sensor outputs the "majority zone" signal.

3. Method according to claim 2,
**characterised in that**
a sufficiently great change in the amplitude (70) is present if the amplitude (70) deviates by less than an amount to be fixed from the target value (74), to which the amplitude (70) is adjusted in the phases in which majority zones (25, 35) are examined with the sensor (10).

4. Method according to one of claims 1 to 3,
**characterised in that**
the sensor (10) receives information via a sensor configuration or via a control input prior to the start of the measurement on whether the amplitude (70) of the measurement signal decreases or increases during the transition from a majority zone (25, 35) to a minority zone (23, 33).

5. Method according to one of claims 1 to 4,
**characterised in that**
the material (11) to be examined is a paper or foil sheet (32) having adhesive strips (34) applied to it or adhesive points, or the material (11) to be examined is a paper or foil sheet (22) having stickers (24) applied thereto.

6. Method according to one of claims 1 to 5,
**characterised in that**
the adjustment of the amplitude (70) to the target value (74) is carried out by an average value of a number of previous measurements of the amplitude (70) being determined, and
if the average value lies above the target value (74), the amplification is decreased by one unit and the adjustment is continued with this amplification and
if the average value lies below the target value (74), the amplification is increased by one unit and the adjustment is continued with this amplification.

7. Method according to one of claims 1 to 6,
**characterised in that**
in situations in which the amplitude (70) assumes a greatest possible value over a time span to be fixed, the sensor (10) assumes that no material (11) is located in the spatial detection region (15) of the sensor (10).

8. Method according to one of claims 1 to 7,
**characterised in that**
"adhesive point", "sticker" and "base material" signals are output by the sensor (10) according to the following:
a) if a material (30) is examined, wherein the amplitude (70) decreases during the transition from the majority zones (35) to the minority zones (33),
a1) the "base material" signal is output if the sensor (10) additionally outputs the "majority zone" signal; and
a2) the "adhesive point" signal is output if the sensor (10) additionally outputs the "minority zone" signal;
b) if a material (20) is examined, wherein the amplitude (70) increases during the transition from the majority zones (25) to the minority zones (23),
b1) the "base material" signal is output if the sensor (10) additionally outputs the "minority zone" signal; and
b2) the "sticker" signal is output if the sensor (10) additionally outputs the "majority zone" signal.

9. Method according to one of claims 1 to 8,
**characterised in that**
a capacitive sensor or an ultrasonic sensor is used as a sensor (10).

10. Method according to one of claims 1 to 9,
**characterised in that**
a barrier sensor or a reflection sensor is used.

## Revendications

1. Procédé de différenciation, par une technique de mesure, de zones de matériau, à savoir de zones majoritaires et de zones minoritaires, d'un matériau de type lame, bande ou feuille, dans lequel
- au moins un capteur (10) est utilisé, lequel relève un signal de mesure présentant une amplitude (70) variable,
- l'amplitude (70) du signal de mesure du capteur (10) varie en fonction entre autres de la zone de matériau se trouvant dans une zone de détection (15) spatiale du capteur (10) et examinée avec le capteur (10),
- que l'amplitude (70) du signal de mesure du capteur (10) est amplifiée avec un amplificateur (45) pouvant être réglé,
- le matériau (11) à examiner est déplacé par rapport au capteur (10) de sorte que les zones majoritaires (25, 35) se trouvent plus fréquemment dans la zone de détection (15) spatiale du capteur (10) en comparaison avec les zones minoritaires (23, 33),
- les zones majoritaires (25, 35) présentent une première épaisseur et une première composition de matériau,
- les zones minoritaires (23, 33) présentent une deuxième épaisseur différente de la première épaisseur et/ou une deuxième composition de matériau différente de la première composition de matériau,
- lors de phases, dans lesquelles des zones majoritaires (25, 35) sont examinées avec le capteur (10), une régulation de l'amplitude (70) à une valeur de consigne (74) est effectuée avec l'amplificateur (45) et le capteur (10) émet un signal « zone majoritaire », et
- dans des situations, dans lesquelles l'amplitude (70) est suffisamment fortement modifiée, le capteur (10) émet un signal « zone minoritaire »,
**caractérisé en ce**
**qu'**une modification suffisamment forte de l'amplitude (70) existe quand l'amplitude (70) s'écarte de la valeur de consigne (74) de plus qu'une grandeur à fixer, à laquelle est régulée l'amplitude (70) lors des phases, dans lesquelles des zones majoritaires (25, 35) sont examinées avec le capteur (10), et
**que**, dans des situations, dans lesquelles le capteur (70) émet le signal « zone minoritaire », la régulation de l'amplitude (70) à la valeur de consigne (74) est désactivée.

2. Procédé selon la revendication 1,
**caractérisé en ce**
**que**, dans des situations, dans lesquelles la régulation est désactivée et dans lesquelles l'amplitude (70) est suffisamment fortement modifiée dans le sens opposé comme dans le cas de la dernière modification la plus récente, qui a donné lieu à la désactivation de la régulation, la régulation est à nouveau activée et le capteur émet le signal « zone majoritaire ».

3. Procédé selon la revendication 2,
**caractérisé en ce**
**qu'**une modification suffisamment forte de l'amplitude (70) existe quand l'amplitude (70) s'écarte de la valeur de consigne (74) de moins qu'une grandeur à fixer, à laquelle l'amplitude (70) est régulée lors des phases, dans lesquelles des zones majoritaires (25, 35) sont examinées avec le capteur (10).

4. Procédé selon l'une quelconque des revendications 1 à 3,
**caractérisé en ce**
**que** le capteur (10) obtient, avant le début de la mesure, par l'intermédiaire d'une configuration du capteur ou par l'intermédiaire d'une entrée de commande, une information pour savoir si l'amplitude (70) du signal de mesure chute ou augmente lors du passage d'une zone majoritaire (25, 35) vers une zone minoritaire (23, 33).

5. Procédé selon l'une quelconque des revendications 1 à 4,
**caractérisé en ce**
**que** le matériau (11) à examiner est une feuille de papier ou une feuille de film (32) avec des bandes adhésives (34) ou emplacements adhésifs appliqués sur celle-ci, ou
**que** le matériau (11) à examiner est une feuille de papier ou de film (22) avec des autocollants (24) appliqués sur celle-ci.

6. Procédé selon l'une quelconque des revendications 1 à 5,
**caractérisé en ce**
**que** la régulation de l'amplitude (70) à la valeur de consigne (74) est effectuée en ce qu'une valeur moyenne d'un nombre de mesures les plus récentes de l'amplitude (70) est définie, et
**que**, quand la valeur moyenne est supérieure à la valeur de consigne (74), l'amplification est réduite d'une unité et la régulation se poursuit avec ladite amplification, et
**que**, quand la valeur moyenne est inférieure à la valeur de consigne (74), l'amplification est augmentée d'une unité et la régulation se poursuit avec ladite amplification.

7. Procédé selon l'une quelconque des revendications 1 à 6,
**caractérisé en ce**
**que**, dans des situations, dans lesquelles l'amplitude (70) adopte une valeur la plus grande possible sur une période à fixer, le capteur (10) part du principe qu'aucun matériau (11) ne se trouve dans la zone de détection (15) spatiale du capteur (10).

8. Procédé selon l'une quelconque des revendications 1 à 7,
**caractérisé en ce**
**que** des signaux « emplacement adhésif », « autocollant » et « matériau de base » sont émis par le capteur (10) selon les critères suivants :
a) quand un matériau (30) est examiné, pour lequel l'amplitude (70) chute lors du passage des zones majoritaires (35) vers les zones minoritaires (33),
a1) le signal « matériau de base » est émis quand le capteur (10) émet par ailleurs le signal « zone majoritaire » ; et
a2) le signal « emplacement adhésif » est émis quand le capteur (10) émet par ailleurs le signal « zone minoritaire » ;
b) quand un matériau (20) est examiné, pour lequel l'amplitude (70) augmente lors du passage des zones majoritaires (25) vers les zones minoritaires (23),
b1) le signal « matériau de base » est émis quand le capteur (10) émet par ailleurs le signal « zone minoritaire » ; et
b2) le signal « autocollant » est émis quand le capteur (10) émet par ailleurs le signal « zone majoritaire ».

9. Procédé selon l'une quelconque des revendications 1 à 8,
**caractérisé en ce**
**qu'**un capteur capacitif ou un capteur à ultrasons est utilisé en tant que capteur (10).

10. Procédé selon l'une quelconque des revendications 1 à 9,
**caractérisé en ce**
**qu'**est utilisé un capteur de barrière ou un capteur de réflexion.
